# EUROPEAN PATENT SPECIFICATION

(11) **EP 3 420 788 B1**
(45) Date of publication and mention of the grant of the patent: **11.12.2019**
(21) Application number: 17179113.0
(22) Date of filing: 30.06.2017
(51) Int. Cl.: A01B 63/22, A01B 63/32

(54) **AGRICULTURAL MACHINE AND METHOD FOR OPERATING AN AGRICULTURAL MACHINE**
LANDWIRTSCHAFTSMASCHINE UND VERFAHREN ZUM BETRIEB EINER LANDWIRTSCHAFTSMASCHINE
MACHINE AGRICOLE ET SON PROCÉDÉ DE FONCTIONNEMENT

(43) Date of publication of application: 02.01.2019
(73) Proprietor: Kverneland Group Les Landes Genusson S.A.S., 85130 Les Landes Genusson (FR)
(72) Inventor: Poulard, Janny, 85600 Saint hilaire de Loulay (FR); Pasquier, Gaëtan, 85110 Saint Cécile (FR)
(74) Representative: Bittner, Thomas L.

(56) References cited:
- US-A- 3 663 032
- US-A- 6 164 385
- US-A1- 2007 023 195
- US-A1- 2015 156 950

## Description

The present invention refers to an agricultural machine and a method for operating an agricultural machine.

### Background

Agricultural machines such as implements to be hitched to a tractor, depending on the type of implement may need adjustment of the working tools provided on a frame of the implement relative to the soil and / or some agricultural product to be engaged with the working tools. For example, for a soil cultivation device there is need for adjustment of the working height / depth of the cultivating working tools in operation.

Document WO 2013 / 178562 A1 discloses an agricultural soil cultivation device which is provided with a hydraulic control system for adjusting the working depth of the working tools in operation. The soil cultivation device comprises a frame, a transport chassis which is arranged such as to be height-adjustable relative to the frame by means of double-acting hydraulic cylinders and which has running wheels, at least one soil roller optionally arranged behind the soil cultivation tools so as to be height-adjustable by means of double-acting hydraulic cylinders, and supporting wheels arranged in the front region of the frame such as to be height-adjustable relative to the frame by means of double-acting hydraulic cylinders.

Document WO 2012 / 125109 A1 refers to an agricultural implement for working soil across. The implement comprises a frame, a first depth maintaining unit comprising a first hydraulic actuator for adjusting the height of the first depth maintaining unit relative to the frame, a second depth maintaining unit comprising a second hydraulic actuator for adjusting the height of the second depth maintaining unit relative to the frame, at least one soil working tool, and a hydraulic system comprising the hydraulic actuators, which is adapted to be connected to means for supplying hydraulic fluid to the hydraulic system.

Document US 2013 / 0068489 A1 refers to an operator-controllable system that allows the operator to make initial field operation settings for the towed implement when hitching the implement to the tractor. Once an initial setup is completed, an operation, the optimal operation settings are automatically maintained throughout the vertical operational range of the working tool.

Document US 2015/0156950 A1 discloses an agricultural implement having agricultural implement for supporting a plurality of gangs of disk blades extending generally laterally relative to a forward travel direction. The implement has carrier frames pivotally connected to wheel assemblies for controlling the height of the carrier frames relative to the ground through hydraulic actuators acting on the wheel assemblies. The hydraulic control unit enables independent and individual control of each actuator. An electronic control unit (ECU) receives signals from a series of inclinometers where each inclinometer signal is compared to the others and the individual actuator operated to bring the actuators into in uniform synchronization.

Document US 6,164,385 A discloses an automatic depth control system. The system includes a Console and remote Controller mounted in the tractor cab, power beyond Valving, and Depth Sensors, either ground contact or non-ground contact, mounted on the frame of the implement. The depth sensing system is provided to sense the actual penetration of an implement tool by determining the height of the implement frame above the ground. A circuit system is provided to receive the depth signal and to signal power beyond valving that will effect hydraulic corrections in order to maintain the select depth penetration. The Console includes an instrument panel having a toggle switch with AUTO, LOCK and MANUAL positions, an LED showing depth of tool penetration and a bargraph depicting variations from a desired depth and flashing lights on the bargraph to show depth corrections in process. The Console also includes a depth window in which no hydraulic correction would be made, a sensitivity switch, depth sensor switches and an UP/REPHASE switch. A remote Controller provides for a five position, mechanical hold toggle switch to provide separate and distinct settings, a rocker switch to vary depth selections in temporary memory, a RUN/HOLD position on existing tool depth penetration, and a SET switch to place settings from temporary to permanent memory. Power beyond valving, incorporating function with either gear, pressure compensated or load sensing hydraulic pumps, is mounted in line with existing hydraulics to provide hydraulic depth selections in either automatic or manual modes.

In document US 3,663,032 A an implement is disclosed which includes pivotally interconnected and wheel supported main and outtrigger frames which can be raised and lowered on the wheels by a plurality of hydraulic cylinders, there being a single cylinder connected between each outrigger frame and its respective wheel and a pair of cylinders connected between the main frame and its wheels with the single cylinders and one of the pair of cylinders being hydraulically connected in series and the pair of cylinders being hydraulically connected in parallel. By employing the pair of parallel cylinders, lift capacity is increased since one of the pair of cylinders operates to raise the main frame while the other of the pair of parallel cylinders passes fluid pressure onto the single cylinders to raise the outrigger frames.

Document US 2007/0023195 A1 refers to a lift system which includes parallel connected cylinder circuits with cylinders mechanically tied together on a first lift wheel arm. The base end of a third cylinder connected to a second lift wheel arm is connected in parallel with the pair of cylinders. The rod end of the third cylinder is constrained for movement with the rod end of a fourth cylinder also connected to the second wheel arm. The rod end of one of the pair of cylinders is connected in series with the base end of the fourth cylinder to thereby constrain the third and fourth cylinders on the second lift wheel arm to move in unison with the pair of cylinders on the first lift wheel arm. Series cylinder circuits move the wing cylinders in unison with the main frame cylinders to keep the implement level. The hydraulic circuit is also plumbed to retract outer wing wheels during wing fold operations to eliminate outer wing wheel interference.

### Summary

It is an object to provide an agricultural machine and a method for operating the agricultural machine which provide for improved operation options with regard to movement control of functional elements such as, for example, working tools to engage with the soil and / or some agricultural product and / or frame elements.

For solving the object, an agricultural machine and a method for operating an agricultural machine according to independent claims 1 and 14, respectively, are provided. Alternative embodiments are disclosed in dependent claims.

Movement of the hydraulic cylinder provides for extending or retracting the hydraulic cylinder. In the process of such movement the one or more pistons of the hydraulic cylinders are moved relative to the cylinder body or barrel which may also be referred to cylinder housing. Movement of the one or more pistons will cause movement of the piston rod relative to the cylinder body. Externally the piston rod may provide a coupling point coupling the force provided by the hydraulic cylinders to some element or part to be located or relocated (moved to a position).

The control system may also be referred to as hydraulic control system.

For movement of the hydraulic cylinders, each of the hydraulic cylinders will be connected to a hydraulic fluid source and a hydraulic fluid pump (supply devices). Such supply devices for providing a pressurized fluid, at least in part, may be provided on a tractor. The hydraulic cylinders connect to the supply by supply lines through which the pressurized fluid is provided to the hydraulic cylinders in operation for movement.

The machine may be provided as an implement. In an alternative embodiment, the supply devices for providing the pressurized fluid, at least in part, may be provided on the implement.

In case of the working tools engaging with the soil the height control may also be referred to as working depth control. For both, height and depth control the vertical position of the working tools is controlled by adjusting the relative position between frame elements.

The control system may be a hydraulic control system applying control adjustment, for example height or depth control, by means of a plurality of hydraulic cylinders driven or operated by a hydraulic control circuit. The hydraulic cylinders may be made of hydraulic cylinders having, for example, a three- or four-chamber design.

The hydraulic cylinders may also be referred to as (hydraulic) actuators configured for moving an element of the machine into different positions.

In an alternative embodiment, synchronization may be provided for adjustment or movement of (hydraulic) cylinders on opposite sides of the agricultural machine, such as an implement, for example, on a front and a rear side or a right and a left side.

There may be more than one position sensors, the sensors provided on different hydraulic cylinders.

The frame may be the frame of an implement to be drawn by or to be hitched to a tractor.

The wording "hydraulic fluid" as used here may be any pressurized fluid which can be applied to operate the hydraulic cylinders. The hydraulic fluid system and the hydraulic fluid pump system are to provide the pressurized fluid to the cylinders for operation.

The synchronization circuit may be configured to synchronize movement of the hydraulic cylinders in terms of at least one of distance of the movement and speed of the movement. For example, if the hydraulic cylinders are fed through the power or supply line connecting the hydraulic cylinders to the hydraulic fluid pump device in operation, because of the synchronization circuit all of the hydraulic cylinders are moved by the same distance, and, optionally, by the same speed. Such synchronization may be achieved or performed by the synchronization circuit independent of different external loads acting on or applied tot the hydraulic cylinders.

In an embodiment, at least or exactly 8 or 10 hydraulic height- / depth-adjustment cylinders may be provided.

The hydraulic cylinders may be provided with one of a three-chamber cylinder design and a four-chamber cylinder design.

Sub-chambers of the hydraulic cylinders are connected to the hydraulic fluid source and the hydraulic fluid pump device through one or more supply lines provided separately from the synchronization circuit. The hydraulic fluid source and / or the hydraulic fluid pump device may be provided as part of the control system or separated from the control system.

The functional elements may comprise at least one of working tools and frame elements of the frame. Working tool, for example, may comprise a tine, a disc, a blade, a knive, a rotor and / or a roller. Frame element may be, for example, any kind of frame, sub-frame, bracket and / or structural parts of the machine.

The agricultural machine may comprise working tools are provided on a first frame element of the frame, the working tools being configured to engage with soil and / or an agricultural product in a plurality of working positions which are controlled by the control system in a control mode, wherein, for locating the working tools in the plurality of working positions, a position of the first frame element relative to a second frame element of the frame is adjustable by means of the control system. The control system may further comprises the hydraulic cylinders configured to adjust the relative position between the first and second frame elements, wherein the hydraulic cylinders each are provided with: a cylinder chamber; a front sub-chamber and a rear sub-chamber both provided in the cylinder chamber and separated by an inner cylinder wall; and a piston rod which is movably extending through a front end of the hydraulic cylinder and the inner cylinder wall and on which a front piston and a rear piston are provided, wherein the front piston is provided in the front sub-chamber and the rear piston is provided in the rear sub-chamber, thereby, the front piston dividing the front sub-chamber, with respect to the inner wall, into a proximal front sub-chamber and a distal front sub-chamber, and the rear piston dividing the rear sub-chamber, with respect to the inner wall, into a proximal rear sub-chamber and a distal rear sub-chamber. In an embodiment, the synchronization circuit may connect the proximal front sub-chamber and the distal front sub-chamber. In an alternative embodiment, the synchronization circuit may connect the proximal front sub-chamber and the proximal rear sub-chamber.

The agricultural machine may comprise at least one of the following: a transport chassis having transport wheels and being adjustable, for example in height, by means of the hydraulic control device; a front gauge wheel provided on a front part of the frame and being adjustable, for example in height, by means of the hydraulic control device; and a ground roller wheel which is optionally to be placed in the rear of working tools and is adjustable, e.g. in height, by means of the hydraulic control device. One or more of the above device elements may be used, for example, for height / depth adjustment of the working tools. One or more of the above device elements may be adjusted at the same time. The device elements may also be referred to as levelling equipment. The adjustment may be done relative to some frame elements carrying some of the working tools.

The agricultural machine may further comprise first hydraulic cylinders and second hydraulic cylinders, for example, front and rear hydraulic cylinders. The first hydraulic cylinders and the second hydraulic cylinders may be provided on different frame part (e.g. front / rear; right / left). The first hydraulic cylinders and the second hydraulic cylinders may be provided on at least one of a first and a second frame element. The first hydraulic cylinders may be configured, for relative position adjustment, to height-adjust the one or more front gauge wheels relative to the frame element on which the working tools are received, such as the first frame element. The second hydraulic cylinders may be configured, for relative position adjustment, to height-adjust the one or more ground roller wheels relative to the frame element carrying the working tools.

The first fluid lines, if the hydraulic cylinders, for example, are provided with a four chamber design, may be connecting the proximal front sub-chambers and the proximal rear sub-chambers of the front hydraulic cylinders and the rear hydraulic cylinders. The second fluid lines separated from the first fluid lines may be connecting the distal front sub-chambers and the distal rear sub-chambers of the front hydraulic cylinders and the rear hydraulic cylinders to the hydraulic fluid pump device.

The agricultural machine may further comprise a hydraulic first control loop, such as a front control loop, configured for working position (e.g. height) control of the functional elements, e.g. the working tools, by the first hydraulic cylinders, and a hydraulic second control loop, such as a rear control loop, configured for position control (e.g. height) of the functional elements, e.g. the working tools, by the second hydraulic cylinders, the hydraulic first control loop being operable separately from the hydraulic second control loop. Controlling of the first hydraulic cylinders and the second hydraulic cylinders may be applied separately for independent position or movement control, such as height / depth control, with regard to frame section(s), for example front or rear frame section(s).

The agricultural machine, which in the alternative embodiments may be provided with or as an implement, may further comprise a draw bar provided on the frame, and a draw bar hydraulic cylinder provided with the hydraulic control system, the draw bar hydraulic cylinder configured, for traction control, to adjust load applied to one or more hitch points of the draw bar when the draw bar is connected to a tractor in the one or more hitch points. In this embodiment traction control is provided in addition to the position control for the functional elements, such as height / depth control. The control system does provide for both, position control and traction control. For the functional elements, e.g. working tools, a working depth in the soil may be kept constant by the operation of the control system, the control system operating both the position control and the traction control. Such controlling is done by applying control signals to the different hydraulic cylinders. A similar operation of a constant working position relative to the soil, but the working tools engaging with some agricultural product, but not with the soil itself, may be applied in such operation situation.

The agricultural machine may further comprise an offset control provided with the control system, the offset control being configured to control an offset between, for example, a front height position applied by the front hydraulic cylinders and a rear height position applied by the rear hydraulic cylinders. In an alternative embodiment, positions on a right and a left side of the frame may be provided with an offset. The front height position may be applied for the hydraulic cylinders for the front gauge wheels. The rear height position may be applied for the hydraulic cylinders assigned to the one or more ground roller wheels. In an alternative embodiment, the front and rear depth control device (e.g. rear roller and front gauge wheels) may be adjusted at the same height relative to the frame. Therefore, the frame is parallel to the soil surface, which is the desired position for work. Depending on working conditions, it may happen that the front and rear depth control device or right and left depth control devices do not have the same carrying capacity. For example, when soil is compact, the front depth control device are running on the soil surface without penetrating into the soil, whereas the rear depth control device are penetrating into the soil which has been preliminary processed by the tillage device in case of a tillage implement. It results in a possible need of an offset adjustment between front and rear depth control device. In a similar way, an offset, in general, may be applied for two different groups of the hydraulic cylinders.

The frame may have at least two frame sections provided adjacent to each other in a direction transverse to a driving direction, and each of the at least two frame sections may be provided with working tools, at least one of the first hydraulic cylinders (e.g. front hydraulic cylinders), and at least one of the second hydraulic cylinders (e.g. rear hydraulic cylinders). In such embodiment there may be more than one ground roller wheels, at least one ground roller wheel assigned to each of the at least two frame sections. For example, there may be three or more frame sections provided adjacent to each other. One or more hydraulic cylinders provided to different frame sections may be operated or adjustable independently for the two or more sections, specifically for applying height control or height-adjustment for the working tools.

The agricultural machine may further comprise a user control terminal functionally connected to the hydraulic control system, the user control terminal configured to receive user input for user setting of control parameters to be applied by the hydraulic control system. By the user control input a setting of one or more hydraulic valves of the control system may be set for controlling positioning of the functional elements such as height / depth control. There may be one or more hydraulic solenoid valve for setting working position. In a similar way, alternatively or in addition, a user input for traction control may be received through the user control terminal. Parameters set by the user, for example, can be: working depth of the implement in cm or inch, offset distance between front and rear depth control device in cm or inch, and force value in N, Kg or other unit, or percent ratio of the max force applied on hitch point for traction control / weight transfer.

The control system may comprise an electronic control unit executing one or software applications, a user interface to display parameters and allow user to enter settings. Depending on user setting, values and sensor inputs sensing the real physical values on the implement, the electronic control unit may deliver a control signal to hydraulic valves or other actuators commonly referred to hydraulic cylinders in the present disclosure to establish the implement in the desired physical situation.

The agricultural machine may further comprise one or more of the following sensor elements: a first pressure sensor configured to detect a load force to the one or more hitch points of the draw bar when the draw bar is connected to the tractor; and a second pressure sensor configured to detect a load force to at least one of depth control element and the front gauge wheel.

The agricultural machine may further comprise a position sensor provided on at least one of the hydraulic cylinders, the position sensor configured to detect position sensor signals for at least one of the front and the rear piston in the cylinder chambers of the hydraulic cylinder. There may be a front position sensor may be provided on one of the front hydraulic cylinders, the front position sensor configured to detect position sensor signals for at least one of the front and the rear piston in the cylinder chamber of the front hydraulic cylinder.The agricultural machine may be further comprising one or more sensor elements having a magneto-restrictive or Hall-effect sensor, a resistive sensor, and / or a photoelectric sensor.

The position sensor can, for example, generate a current, or a voltage, or a pulse frequency, when analog sensors, proportional to the measures position, or a digital message indication the position when digital sensor. This information may be communicated to the electronic control unit via a data bus or any other communication means. The electronic control unit may generate, in response, a current, or a voltage, or a pulse frequency, when analog electro valves, proportional to the desired position, or a digital message indication of the desired position when digital electro valves. Therefore, the control system may be implementing real time adjustment of the position of the hydraulic cylinders to a desired position.

The working tools may comprise soil cultivating tools. Regarding to such embodiment, the frame with the soil cultivating tools may be provided with an implement being an agricultural soil cultivation machine to be hitched to a tractor. However, the technologies disclosed here may apply to other types of implement such as windrower, seeder, mower, disc harrow, tine harrow, sprayer, and plough. With regard to the alternative embodiments, working tools and / or frame elements may be positioned in synchronization by the control system.

The principles of the control system may be applied to implements of different type for controlling working or relative positions of functional elements of the implement such as frame elements and / or working tools of the implement. By the control system, in general, a plurality of functional elements may be controlled. In an embodiment it provides for the option for synchronized control with regard to the plurality of functional elements such as elements of the frame and / or working tools. In general, the controlling may refer to controlling adjusting a position of a functional element in relation to another functional element of the implement.

With regard to an alternative embodiment having the hydraulic cylinders of the control system provided with the four-chamber design, the synchronization circuit comprising one or more synchronization (pressurized) fluid lines may connect the distal front sub-chamber and the proximal front sub-chamber of the hydraulic cylinders. There may be a communication valve assigned to each of the hydraulic cylinders and connecting the the distal front sub-chamber and the proximal front sub-chamber of the hydraulic cylinder.

With regard to an alternative embodiment having the hydraulic cylinders of the control system provided with the three-chamber design, the synchronization circuit comprising one or more synchronization (pressurized) fluid lines may connect in a series the front sub-chamber and the proximal rear sub-chamber of the hydraulic cylinders. In the series of hydraulic cylinders, for adjacent hydraulic cylinders connected to each other the front sub-chamber of one of the two hydraulic cylinders may be connected to the proximal rear sub-chamber of the other hydraulic cylinder.

For example, the movement of the cylinder rod in one of the hydraulic cylinders may generate a fluid volume variation in the proximal front sub-chambers and the distal front sub-chamber which is communicated to the next hydraulic cylinders via the synchronization circuit. The synchronization circuit may be arranged as a serial connection between the hydraulic cylinders for which the movement is to be synchronized together. For example, the proximal front sub-chambers of a first hydraulic cylinder is connected to the distal front sub-chamber of a second hydraulic cylinder following the first hydraulic cylinder. Further, the proximal front sub-chambers of the second hydraulic cylinder is connected to the distal front sub-chamber of a third hydraulic cylinder following the second hydraulic cylinder.

There may be synchronizing the movement of the hydraulic cylinders in terms of at least one of distance of the movement and speed of the movement. For example, if the hydraulic cylinders are fed through the power or supply line connecting the hydraulic cylinders to the hydraulic fluid pump device, because of the synchronization circuit, all of the hydraulic cylinders may be moving the same distance and / or with the same speed of movement, such as movement of the piston rod. Such synchronization may be achieved or performed by the synchronization circuit independent of different external loads or forces acting on or applied tot the hydraulic cylinders. The hydraulic cylinders may acting (moving) under or against different external load applied to the hydraulic cylinders, for example, to the piston rod. Still, the synchronization circuit connecting to all of the hydraulic cylinders will provide for synchronized movement of all of the hydraulic cylinders connected to the synchronization circuit.

The alternative embodiments described above with regard to the agricultural machine may apply to the method of operating the agricultural machine *mutatis mutandis.*

### Description of embodiments

Following, further embodiments are described with reference to figures. In the figures, show:
- Fig. 1: a schematic block diagram of components of a control system provided in an agricultural system;
- Fig. 2: a schematic representation of an implement provided as a soil cultivating device (cultivator) in a top view;
- Fig. 3: a schematic representation of the soil cultivating device hitched to a tractor in a side view;
- Fig. 4: a schematic representation of another soil cultivating device in a side view;
- Fig. 5: a schematic representation of an alternative control system;
- Fig. 6: a schematic representation of a hydraulic cylinder comprising sub-chambers;
- Fig. 7: a schematic representation of a hydraulic system comprising a plurality of hydraulic cylinders; and
- Fig. 8: a schematic representation of an alternative control system to be applied to an implement.

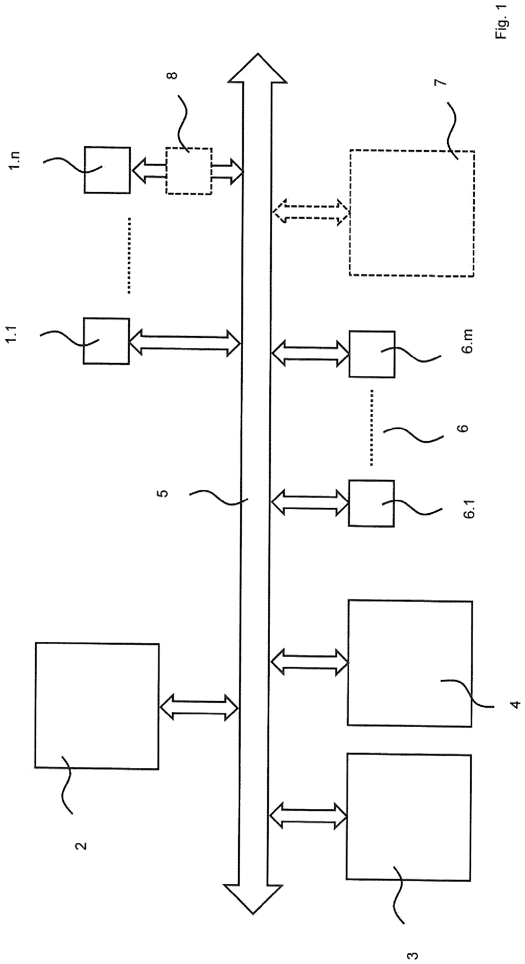

Fig. 1 shows a schematic block diagram of components of a control system provided for use in an agricultural system comprising a tractor and an implement (agricultural machine) drawn by the tractor. The implement is provided with working tools which in operation will engage the ground and / or some agricultural product while the tractor is pulling the implement over the field. The implement, for example, may be a cultivator, a windrower, a seeder, a mower, a disc harrow, a tine harrow or a plough.

The alternative aspects of the present disclosure may also apply to implements for which the working elements do not engage with the soil such as a sprayer. Still, functional elements are to be located / relocated into different working positions in operation of the implement.

The working tools, as it is known as such in the art, may be moved between a working position in which the working tools are engaging with the ground and / or an agricultural product, and a non-working position in which the working tools are disengaged from the ground and / or the agricultural product. Usually, there are more than one working position. There may a plurality of non-working positions, the working tools in each of the non-working positions being disengaged from the ground and / or the agricultural product. At least some of the non-working positions may be referred to as transport positions. Such one or more transport positions may be applied to the working tools for pulling the implement either over the field or on a street in a transport situation.

The arrangement shown in Fig. 1 is provided with a sensor arrangement 1 comprising one or more sensor elements 1.1, ..., 1.n (n ≥ 2). The sensor elements 1.1, ..., 1.n are each configured to detect one or more measurement components or parameters (measurement signals) such as force, pressure, angle and / or speed. The sensor elements 1.1, ..., 1.n are connected to a control unit 2 which is to receive and process sensor or measurement signals. According to the exemplary embodiment in Fig. 1 the control unit 2 is connected to a display unit 3 and memory unit 4. Through the display unit 3 information signals may be displayed or outputted to the user of the agricultural system, for example, the driver of the tractor. The display unit 3 may be provided in a user terminal located, for example, in the tractor cab. The user terminal may comprise at least one of the control unit 2 and the memory unit 4, at least in part.

In the memory unit 4 data may be stored by the control unit 2, for example, log data which provide information about the operation of the agricultural machine. Such log data provided in one or more log data files may be retrieved to derive statistic data or information about the operation of the agricultural machine by the control unit 2.

In an alternative embodiment, there may be an implement free of the sensor elements 1.1, ..., 1.n.

The components of the control system are, specifically for data transmission, functionally connected to a control bus 5 of the agricultural machine such as a CAN bus. For example, the display unit 3 and / or the memory unit 4 may be connected to the control unit 2 directly, thereby, establishing data transmission not through the control bus 5, but direct data exchange.

Referring still to Fig. 1, an arrangement of hydraulic cylinders 6 comprising a plurality of hydraulic cylinders 6.1, ..., 6.m (m ≥ 2). One or more of the sensor elements 1.1, ..., 1.n may be provided with one or more of the hydraulic cylinders 6.1, ..., 6.m.

There may be one or more additional components 7 provided with the control system of the agricultural system. One or more of the sensor elements 1.1, ..., 1.n may be assigned a local control unit 8 which, for example, may implement controlling of the respective sensor element in the process of detecting measurement signals. Also, the local control unit 8, for the assigned sensor element, may control data transmission through the control bus 5.

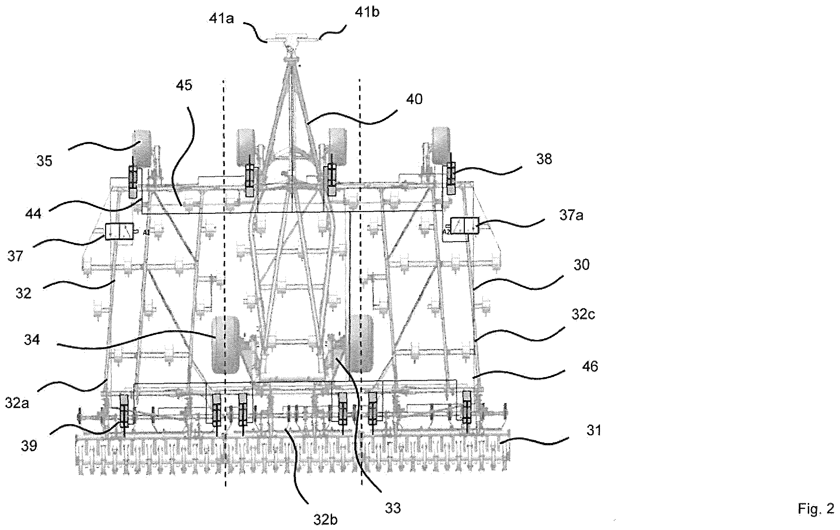

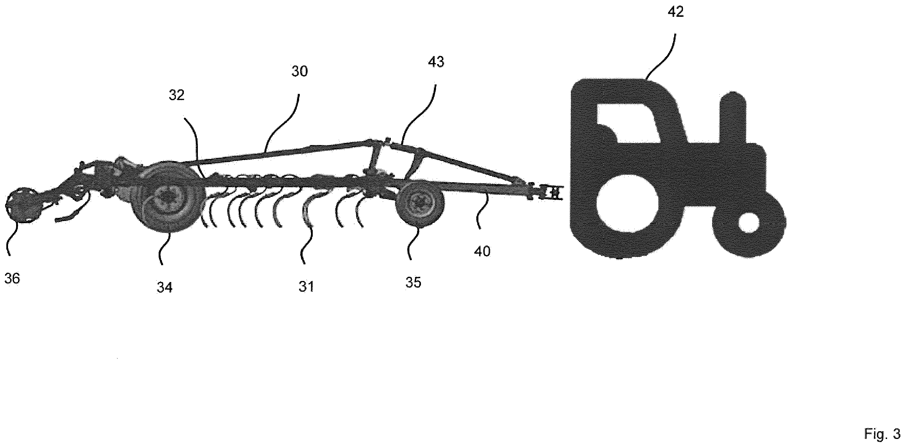

While the tractor is pulling the implement over the field measurement signals may be detected by the sensor elements 1.1, ..., 1.n which, for example, allow to calculate or determine a draft or pull force which is applied to the implement through a draw bar (see Fig. 2 and 3).

Fig. 2 to 4 show an implement which is a soil cultivating device 30 (cultivator) in a top view and side views. Cultivation is an intensive job that requires power to move the soil and mix it properly. A nice finish with a perfect levelled soil is also requested to facilitate the job for the other agricultural equipment's and ensuring good seed growth. The "open windows" for a perfect work can be limited depending of soil conditions and weather conditions. The soil cultivating machine has to deliver the best performances and being efficient for cost establishment.

Following, in an exemplary embodiment reference is made to the soil cultivating device 30. However, principles of the disclosure may refer to other implements as well such as windrower, seeder, mower, disc harrow, tine harrow, sprayer, and plough.

The soil cultivating device 30 is provided with a plurality of working tools 31 which, for engaging with the soil, are movable between a plurality of working positions in which the working tools 31 are engaging with the ground or soil. In addition, the working tools 31 are movable in at least one non-working position in which the working tools 31 are disengaged from the ground / soil for transportation.

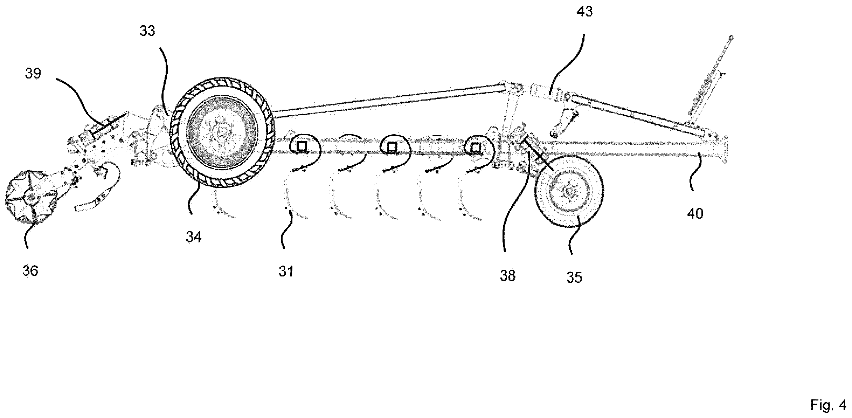

The soil cultivating device 30 comprises a frame 32 on which the working tools 31 are provided. There is a transport chassis 33 having transport wheels 34 at being adjustable in height relative to the frame 32. Front gauge wheels 35 are provided on the front part of the frame 32. The front gauge wheels 35 are adjustable in height relative to the frame 32. Further, there are ground roller wheels 36 (see Fig. 3 and 4) to be placed in the rear of the working tools 31. The ground roller wheels 36 are adjustable in height relative to the frame 32.

The soil cultivating device 30 is provided with three frame sections 32a, 32b, 32c.

A control system 37 comprising a hydraulic block 37a is provided with the soil cultivating device 30 for controlling height / depth of the working tools 31 relative to the soil and / or some agricultural product to be engaged with the working tools 31. Such height / depth control which may also be referred to leveling control is done by adjusting, relative to the frame, the position of at least one of the following: the transport chassis 33 with the transport wheels 34, the front gauge wheels 35, and the ground roller wheels 36. In addition, traction control may be applied.

In an alternative embodiment, as an example, the transport chassis 33 and some other frame element of the frame 32, the other frame element carrying the working tools 31, may provide for two frame elements (first, second) for which, by the control system 37, relative position may be adjusted.

The hydraulic bloc 37a may be operated for offsetting the front gauge wheels 35 and / or reset the control system 37.

The control system 37 comprises front hydraulic cylinders 38 and rear hydraulic cylinders 39 which may be providing an exemplary embodiment of the plurality of hydraulic cylinders 6.1, ..., 6.m. In the embodiment shown, each of the frame sections 32a, 32b, 32c is provided with at least one of the front hydraulic cylinders 38 and at least one of the rear hydraulic cylinders 39. In the embodiment shown, the front hydraulic cylinders 38 and the rear hydraulic cylinders 39 are provided with a four-chamber design.

The soil cultivating device 30 comprises a drawbar 40 provided with hitch points 41a, 41b for hitching the soil cultivating device 30 to a tractor 42 (see Fig. 3). Alternatively, there may be a single hitch point.

The drawbar 40 is assigned a drawbar hydraulic cylinder 43 to be adjusted for traction control, the traction control providing increased or reduced force to the hitch points 41a, 41b.

The control system 37 comprises first and second fluid control lines 44, 45 which are separated. The first and second fluid control lines 44, 45 provide a pressurized fluid for movement of the hydraulic cylinders of the control system 37, thereby, extending and retracting the hydraulic cylinders. The pressurized fluid in the first and second fluid control lines 44, 45 is provided by a hydraulic pump (not shown) which may be located on the tractor. The hydraulic pump may be provided as part of the control system 37 or separated from the control system 37.

Further, there is a synchronization circuit 46 comprising fluid lines connecting to all of the hydraulic cylinders.

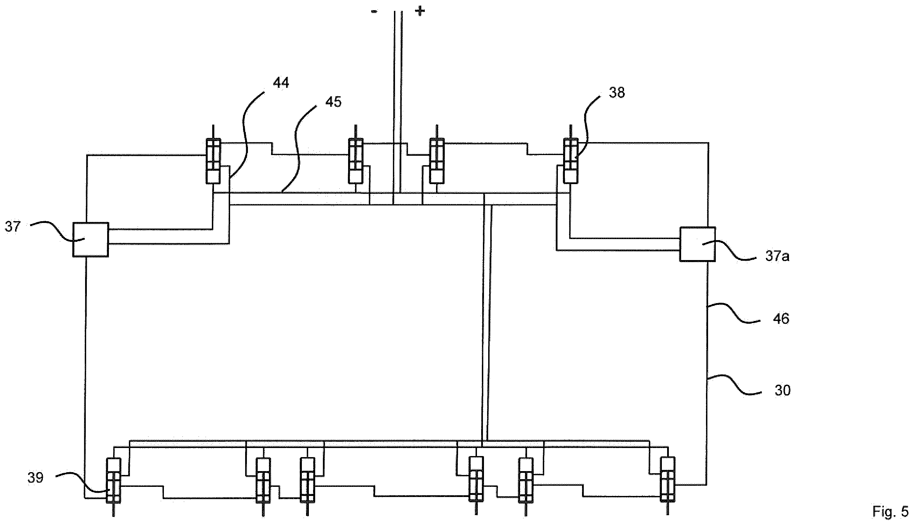

Fig. 5 shows a schematic representation of an alternative embodiment of the control system 37. The control system 37 has been described by reference to the exemplary embodiment in which it is provided on the soil cultivating device 30. However, it may be applied to other implements for controlling working positions of functional elements of the implement such as frame elements and / or working tools of the implement. By the control system 37, in general, a plurality of functional elements may be controlled. In an embodiment it provides for the option for synchronized control with regard to the plurality of functional elements such as elements of the frame and / or working tools.

In the embodiment shown, the front hydraulic cylinders 38 and the rear hydraulic cylinders 39 are provided with a four-chamber design.

In an alternative embodiment, frame elements such as sections of a boom of a sprayer may be may be controlled by the control system of Fig. 5.

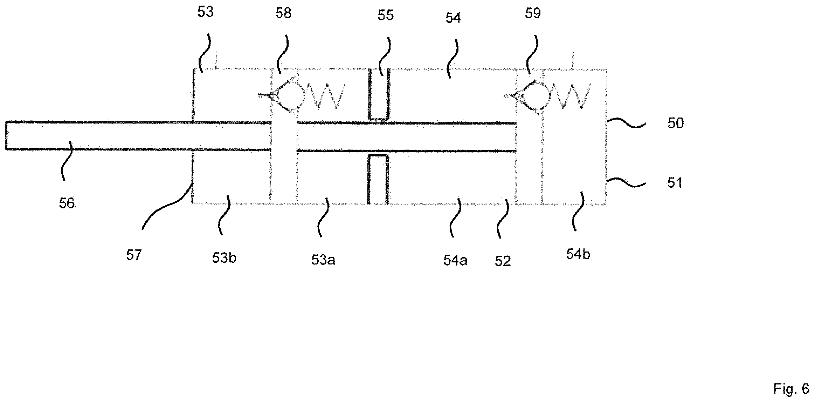

Fig. 6 shows a schematic representation of a hydraulic cylinder 50 which may also be referred to as tandem hydraulic cylinder and which is provided with a housing 51 in which a cylinder chamber 52 is received. The hydraulic cylinder 50 may be applied for at least one or all of the hydraulic cylinders in the control system 37. The cylinder chamber 52 is provided with a front sub-chamber 53 and a rear sub-chamber 54. The front sub-chamber 53 and the rear sub-chamber 54 are separated by a cylinder wall 55. A piston rod 56 is extending through the cylinder wall 55 and a front wall 57. A first and a second piston 58, 59 are provided on the piston rod 57. The first and second piston 58, 59, with respect to the cylinder wall 55, are dividing the front and the rear sub-chamber 53, 54 into a proximal front sub-chamber 53a and a distal front sub-chamber 53b, and a proximal rear sub-chamber 54a and a distal rear sub-chamber 54b. Therefore, the hydraulic cylinder 50 is provided with a design which may be referred to four-chamber (cylinder) design.

The front sub-chamber 53 and the rear sub-chamber 54 are connected in parallel to a fluid pressurized circuit and are used to develop the force on elements functionally connected to the piston rod 56. The proximal rear sub-chamber 54a and the distal rear sub-chamber 54b are used to produce a force thanks to the fluid pressure supply. The proximal front sub-chamber 53a and the distal front sub-chamber 53b may be used for synchronization with other hydraulic cylinders via the fluid volume variation resulting of the extension or retraction of the hydraulic cylinder.

The soil cultivating device 30 has been designed to provide best working quality with high output, while ensuring the lowest costs of use. For that reason, the depth and levelling adjustments may be directly controlled from the tractor cab by the user terminal such as an ISOBUS Terminal. In addition, the depth adjustment may be coupled with traction control to save, for example, fuel. An automatic overload protection may optionally be applied to the machine frame to avoid any downtime operations.

The depth or levelling control for the trailed cultivator 30, optionally combined with traction control, is aiming at less time for adjusting the machine and higher working speed.

The driver of the tractor 42 can set easily the depth of the working tools 31 and the height of the levelling equipment on the user terminal. Automatically the system will adjust all the hydraulic cylinders. A front / rear depth correction can be done at any time depending of soil conditions.

The traction control comprising the drawbar hydraulic cylinder 43 is configured to transfer some weight from the front gauge wheels 35 to the tractor 42 coupling in order to give more grip and traction to the tractor 42. The tractor 42 and the cultivator 30 have always the most efficient synergy: this results in fuel consumption reduction, avoids need of too much extra weight on the tractor 42, and prevents tire wearing by slipping control and avoid soil compaction.

The working depth may be set by the user through user input received in the user terminal by the driver. The user can adjust the load transfer which shall be provided on the tractor. If the user puts 100%, there will be nearly no weight on front gauge wheels 35. The weight reported to the tractor 42 by the sensors may be, for example, close to about 1,8 tons in the heaviest configuration. Or the user may input 0%, following, the weight transfer to the tractor 42 will be 0 kg (to avoid letting wheels tracks if fluffy soil).

The driver can also adjust the maximum height of the transport wheels to save time in the headlands for lifting / lowering the machine.

The customer can adjust the position of the rear levelling device from the cab during driving depending of conditions.

The horizontal position (attitude) is set by the driver from the cab as this adjustment will depend on one or more of the following aspects: the soil conditions (moisture content, soil texture, etc.), the soil structure (first pass, second pass, etc...), and the roller type, tire pressure. The control system will adjust automatically the working depth position, for example, by adjusting the height of the front gauge wheels 35 according to the attitude preset value.

The working depth is adjusted by applying the hydraulic control as outlined above. For example, only two hydraulic cylinders each with a position sensor may be provided to manage, for example, a plurality of ten hydraulic depth cylinders. A master slave system may be implemented in the control system described above.

In hilly conditions, by the traction control the pressure in drawbar hydraulic cylinder 43 is constantly adjusted to maintain always the selected force at the hitch points 41a, 41b.

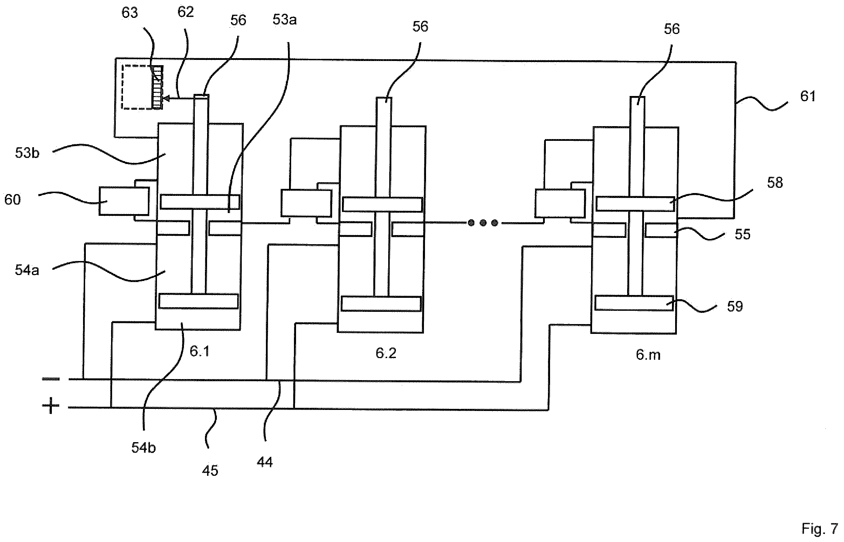

Fig. 7 shows a schematic representation of the plurality of hydraulic cylinders 6.1, ..., 6.m which may be provided in the control system 37. Fig. 7 shows a design of the control system 37 similar to the design in Fig. 5.

Each of the hydraulic cylinders 6.1, ..., 6.m is having a design which may be referred to as four-chamber design and is assigned a communication valve 60. One or more communication valves such as communication valve 60 are optional. The communication valve 60 is missing in the design in Fig. 5 which, in terms of other design aspects, is similar to the design on Fig. 7. The communication valve 60 may be provided, for example, for making it easier the fill in of the circuit with hydraulic fluid (pressurized fluid) after assembly, and / or, resynchronizing the position of the hydraulic cylinders 6.1, ..., 6.m when placing them in fully extended or retracted position, ex resynchronization may be needed to face internal leakages after several hours of work.

The fluid lines 44, 45 are use respectively to retract and extend the hydraulic cylinders 6.1, ..., 6.m when a fluid under pressure is supplied. The fluid pressure into the proximal rear sub-chambers 54a and the distal rear sub-chamber 54b generate a force and the movement of the cylinder rod 57.

There is a synchronization circuit 61 providing synchronization functionality similar to the synchronization circuit 46 in Fig. 2. The movement of the cylinder rod 57 generates a fluid volume variation in the proximal front sub-chambers 53a and the distal front sub-chamber 53b which is communicated to the next hydraulic cylinders via the synchronization circuit 61. The synchronization circuit 61 is arranged as a serial connection between the hydraulic cylinders 6.1, ..., 6.m for which the movement is synchronized together. For example, the proximal front sub-chambers 53a of the hydraulic cylinder 6.1 is connected to the distal front sub-chamber 53b of the following hydraulic cylinder 6.2. Further, the proximal front sub-chambers 53a of the hydraulic cylinder 6.2 is connected to the distal front sub-chamber 53b of the following hydraulic cylinder 6.3.

When the proximal front sub-chambers 53a and the distal front sub-chamber 53b of the different hydraulic cylinders 6.1, ..., 6.m have the same cylinder volume, it results that the fluid volume variation of the proximal front sub-chambers 53a must be equal to fluid volume variation of the distal front sub-chamber 53b of the next hydraulic cylinder. It results in a synchronized movement of the different hydraulic cylinders 6.1, ..., 6.m.

A master-slave operation principle may be applied for the use of the serial synchronization circuit 61, thereby, in an alternative embodiment, also allowing the synchronized movement of the pistons in the hydraulic cylinders 6.1, ..., 6.m by the fluid volume exchange from one to the other cylinder. Indeed, the chambers of the hydraulic cylinders 6.1, ..., 6.m have similar volumes. The system thus may combine advantages of a parallel connection to develop forces and advantage of a serial connection to synchronize piston movements in the hydraulic cylinders 6.1, ..., 6.m, thereby, for example, synchronizing level or height / depth adjustment.

In the embodiment of Fig. 7, the synchronization circuit 61 is connected to the proximal front sub-chamber 53a and the distal front sub-chamber 53b, for the hydraulic cylinders 6.1, ..., 6.m. The proximal rear sub-chamber 54a and the distal rear sub-chamber 54b are connected to the fluid lines 44, 45 for each of the hydraulic cylinders 6.1, ..., 6.m,. A similar design is shown in Fig. 5.

In the alternative embodiment shown in Fig. 2, if the hydraulic cylinders are provided with a design for which an example is shown in Fig. 6, the synchronization circuit 61 is connected to the proximal front sub-chamber 53a and the proximal rear sub-chamber 54a. The distal front sub-chamber 53b and the distal rear sub-chamber 54b are connected to the fluid lines 44, 45 providing the pressurized fluid for cylinder movement.

The hydraulic cylinder 6.1 is provided with a position sensor 62 assigned to a position indicating scale 63. In an alternative embodiment, there may be a scale or another device for measuring the position of the hydraulic cylinder depending on construction variant. One or more position sensors may be provided with at least one of the front hydraulic cylinders 38 and the rear hydraulic cylinders 39.

Based on the position sensor signal, the control system may displays in real time the position value of working depth, or other corresponding setting, on the user interface. When the position sensor signal is different from the user setting received before, the control system may generate a message on the control bus 5 to drive the control valve and thus place the hydraulic cylinders 6.1, ..., 6.m in the desired position.

The use of a parallel power circuit (fluid supply ports "+" and "-") supply a pressurized hydraulic fluid to the system and generate the force developed by the hydraulic cylinders 6.1, ..., 6.m. The pressure into this circuit will be the average pressure generated by the different loads on the system. The fluid supply ports are to be connected to a hydraulic fluid source and / or pump.

The synchronization of the hydraulic cylinders 6.1, ..., 6.m using serial connection between them (e.g., master / slave arrangement) may provide the advantage of avoiding pressure accumulation related to each cylinder external force. The number of hydraulic cylinders 6.1, ..., 6.m is then not limited, and their section can be optimized to the necessary force to develop. The additional sub-chambers are to produce the force in a common double acting cylinder. The 1 to m additional chambers, of similar volumes, are used to synchronize the position (moving) of the 1 to m hydraulic cylinders together, using the fluid volume exchange.

The controlling technology for adjusting height / depth of working tools exemplary described with reference to a soil cultivating machine may, however, be applied to other types of implement such as windrower, seeder, mower, disc harrow, tine harrow, sprayer, and plough.

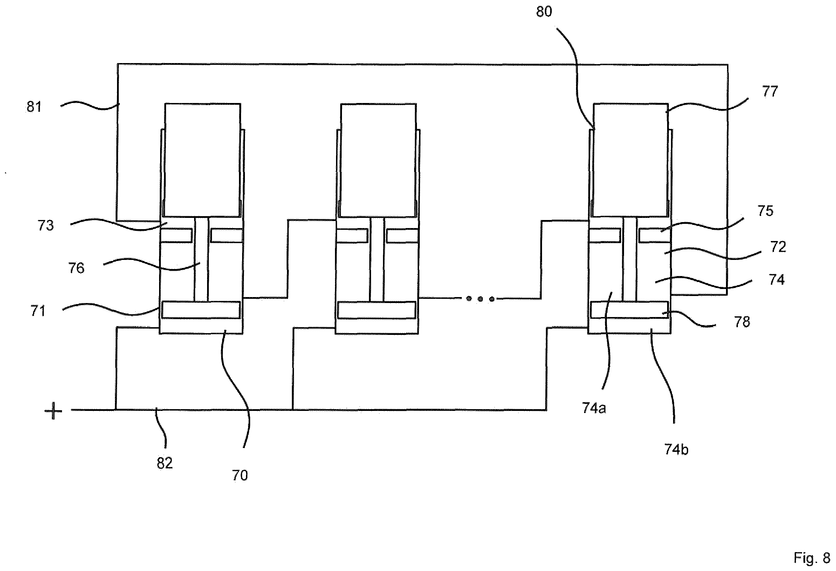

Fig. 8 shows a schematic representation of an alternative arrangement of a plurality of hydraulic cylinders 70 each having a design which may be referred to as three-chamber design. For the hydraulic cylinders 70 there is a housing 71 in which a cylinder chamber 72 is received. The hydraulic cylinder 71 may be applied for at least one or all of the hydraulic cylinders in the control system 37. The cylinder chamber 72 is provided with a front sub-chamber 73 and a rear sub-chamber 74. The front sub-chamber 73 and the rear sub-chamber 74 are separated by a cylinder wall 75. A piston rod 76 is extending through the cylinder wall 75. A first and a second piston 77, 78 are provided on the piston rod 76. The second piston 78, with respect to the cylinder wall 75, is dividing the rear sub-chamber 74 into a proximal front sub-chamber 74a and a distal front sub-chamber 74b. The first piston 77 is extending through a front wall 80.

In the alternative embodiment in Fig. 8 the front sub-chamber 73 is provided with a single chamber design, while the rear sub-chamber 74 is provided with a two sub-chamber design, namely the proximal and the distal rear sub-chambers 74,a, 74b. Therefore, the plurality of hydraulic cylinders 70 may as be referred to as having a three-chamber cylinder design.

A serial synchronization circuit 81 allows synchronized movement of the pistons in the hydraulic cylinders 70 by the fluid volume exchange from one to the other cylinder, for example, in a master-slave design for the cylinders. Indeed, the chambers of the hydraulic cylinders 70 have similar volumes. The system thus may combine advantages of a parallel connection to develop forces and advantage of a serial connection to synchronize piston movements in the hydraulic cylinders 70, thereby, for example, synchronizing level or height / depth adjustment and / or movement of other functional elements of the implement.

A pressurized fluid such as a hydraulic fluid is provided through a line 83 for operating the hydraulic cylinders 70.

## Claims

1. An agricultural machine, comprising
- a frame (32);
- a control system (37) having a plurality of hydraulic cylinders (38, 39; 6.1, ..., 6.m); and
- a plurality of functional elements provided on the frame (32) and movable in working positions by the plurality of hydraulic cylinders (38, 39; 6.1, ..., 6.m);
wherein
- the control system (37) comprises a synchronization circuit (46; 61; 81) connecting to each of the plurality of hydraulic cylinders (38, 39; 6.1, ..., 6.m; 70) from the plurality of hydraulic cylinders (38, 39; 6.1, ..., 6.m; 70), the synchronization circuit (46; 61; 81) configured to synchronize movement of all of the plurality of hydraulic cylinders (38, 39; 6.1, ..., 6.m; 70);
- **characterized in that** the sub-chambers of the hydraulic cylinders (38, 39; 6.1, ..., 6.m; 70) are connected to the hydraulic fluid source and the hydraulic fluid pump device through one or more supply lines (44, 45) provided separately from the synchronization circuit (46; 61; 81).

2. The agricultural machine according to claim 1, wherein the hydraulic cylinders (38, 39; 6.1, ..., 6.m; 70) are provided with one of a three-chamber cylinder design and a four-chamber cylinder design.

3. The agricultural machine according to at least one of the preceding claims, wherein the functional elements comprises at least one of working tools (31) and frame elements of the frame (32).

4. The agricultural machine according to claim 3 wherein the working tools (31) are provided on a first frame element of the frame (32), the working tools (31) being configured to engage with soil and / or an agricultural product in a plurality of working positions which are controlled by the control system (37) in a control mode, wherein, for locating the working tools (31) in the plurality of working positions, a position of the first frame element relative to a second frame element of the frame (32) is adjustable by means of the control system (37);
wherein the control system (37) comprises
- the hydraulic cylinders (38, 39; 6.1, ..., 6.m; 70) configured to adjust the relative position between the first and second frame elements, wherein the hydraulic cylinders (38, 39; 6.1, ..., 6.m; 70) each are provided with
- a cylinder chamber;
- a front sub-chamber and a rear sub-chamber both provided in the cylinder chamber and separated by an inner cylinder wall; and
- a piston rod which is movably extending through a front end of the hydraulic cylinder and the inner cylinder wall and on which a front piston and a rear piston are provided, wherein the front piston is provided in the front sub-chamber and the rear piston is provided in the rear sub-chamber, thereby, the front piston dividing the front sub-chamber, with respect to the inner wall, into a proximal front sub-chamber and a distal front sub-chamber, and the rear piston dividing the rear sub-chamber, with respect to the inner wall, into a proximal rear sub-chamber and a distal rear sub-chamber.

5. The agricultural machine according to at least one of the preceding claims, further comprising at least one of
- a transport chassis having transport wheels (34) and being adjustable in height by means of the control system (37);
- a depth control element provided on a front part of the frame (32) and being adjustable by means of the control system (37);
- a front gauge wheel (35) provided on a front part of the frame (32) and being adjustable by means of the control system (37); and
- a ground roller (36) wheel which is optionally to be placed in the rear of the working tools (31) and is adjustable in height by means of the control system (37).

6. The agricultural machine according to at least one of the preceding claims, further comprising first hydraulic cylinders and second hydraulic cylinders.

7. The agricultural machine according to claim 6, further comprising
- a hydraulic first control loop configured for working position control of the functional elements by the first hydraulic cylinders; and
- a hydraulic second control loop configured for working position control of the functional elements by the second hydraulic cylinders, the hydraulic second control loop being operable separately from the hydraulic first control loop.

8. The agricultural machine according to at least one of the preceding claims, further comprising
- a draw bar (40) provided on the frame (32); and
- a draw bar hydraulic cylinder provided with the control system (37), the draw bar hydraulic cylinder configured, for traction control, to adjust load applied to one or more hitch points of the draw bar (40) when the draw bar (40) is connected to a tractor (42) in the one or more hitch points.

9. The agricultural machine according to at least one of the preceding claims, further comprising an offset control provided with the control system (37), the offset control being configured to control an offset between a front height position applied by the front hydraulic cylinders (38) and a rear height position applied by the rear hydraulic cylinders (39).

10. The agricultural machine according to at least one of the preceding claims, referring to claims 3 and 6, wherein
- the frame (32) is having at least two frame sections provided adjacent to each other in a direction transverse to a driving direction; and
- each of the at least two frame sections is provided with the working tools (32), at least one of first hydraulic cylinders, and at least one of the second hydraulic cylinders.

11. The agricultural machine according to at least one of the preceding claims, further comprising a user control terminal functionally connected to the control system (37), the user control terminal configured to receive user input for user setting of control parameters to be applied by the control system (37).

12. The agricultural machine according to at least one of the preceding claims, further comprising one or more of the following sensor elements:
- a first pressure sensor configured to detect a load force to the one or more hitch points of the draw bar (40) when the draw bar (40) is connected to the tractor (42); and
- a second pressure sensor configured to detect a load force to at least one of depth control element and the front gauge wheel (35).

13. The agricultural machine according to at least one of the preceding claims, further comprising a position sensor provided on at least one of the hydraulic cylinders (38, 39; 6.1, ..., 6.m; 70), the position sensor configured to detect position sensor signals for at least one of the front and the rear piston in the cylinder chambers of the hydraulic cylinder.

14. A method for operating an agricultural machine having
- a frame;
- a control system (37) having a plurality of hydraulic cylinders (38, 39; 6.1, ..., 6.m); and
- a plurality of functional elements provided on the frame (32) and movable in working positions by the plurality of hydraulic cylinders (38, 39; 6.1, ..., 6.m);
wherein
- the control system (37), by a synchronization circuit (46; 61; 81) connecting to each of the plurality of hydraulic cylinders (38, 39; 6.1, ..., 6.m; 70) from the plurality of hydraulic cylinders (38, 39; 6.1, ..., 6.m; 70), is synchronizing movement of all of the plurality of hydraulic cylinders (38, 39; 6.1, ..., 6.m; 70); and
- providing pressurized fluid to sub-chambers of the hydraulic cylinders through one or more supply lines, the one or more supply lines connecting the sub-chambers to the hydraulic fluid source and the hydraulic fluid pump device and being provided separately from the synchronization circuit.

## Patentansprüche

1. Landwirtschaftliche Maschine, umfassend
- einen Rahmen (32);
- ein Steuersystem (37) aufweisend eine Mehrzahl von Hydraulikzylindern (38, 39; 6.1, ..., 6.m); und
- eine Mehrzahl von Funktionselementen bereitgestellt auf dem Rahmen (32) und beweglich in Arbeitspositionen durch die Mehrzahl von Hydraulikzylindern (38, 39; 6.1, ..., 6.m);
wobei
- das Steuersystem (37) eine Synchronisierungsschaltung (46; 61; 81) umfasst, welches mit jedem der Mehrzahl von Hydraulikzylindern (38, 39; 6.1, ..., 6.m; 70) aus der Mehrzahl von Hydraulikzylindern (38, 39; 6.1, ..., 6.m; 70) verbunden ist, wobei die Synchronisierungsschaltung (46; 61; 81) dafür eingerichtet ist, dass sie die Bewegung sämtlicher der Mehrzahl von Hydraulikzylindern (38, 39; 6.1, ..., 6.m; 70) synchronisiert;
- **dadurch gekennzeichnet, dass** die Unterkammern der Hydraulikzylinder (38, 39; 6.1, ..., 6.m; 70) durch eine oder mehrere Versorgungsleitungen (44, 45), die getrennt von der Synchronisierungsschaltung (46; 61; 81) bereitgestellt sind, mit der Hydraulikfluidquelle und der Hydraulikflüssigkeitspumpvorrichtung verbunden sind.

2. Landwirtschaftliche Maschine nach Anspruch 1, wobei die Hydraulikzylinder (38, 39; 6.1, ..., 6.m; 70) entweder mit einer Dreikammerzylinderausführung oder einer Vierkammerzylinderausführung versehen sind.

3. Landwirtschaftliche Maschine nach mindestens einem der vorstehenden Ansprüche, wobei die Funktionselemente Arbeitswerkzeuge (31) und/oder Rahmenelemente des Rahmens (32) umfassen.

4. Landwirtschaftliche Maschine nach Anspruch 3, wobei die Arbeitswerkzeuge (31) an einem ersten Rahmenelement des Rahmens (32) bereitgestellt sind, wobei die Arbeitswerkzeuge (31) dafür eingerichtet sind, in einer Mehrzahl von Arbeitspositionen, die durch das Steuersystem (37) in einem Steuermodus gesteuert werden, in die Erde und/oder ein landwirtschaftliches Produkt einzugreifen, wobei zum Anordnen der Arbeitswerkzeuge (31) in der Mehrzahl von Arbeitspositionen eine Position des ersten Rahmenelements relativ zu einem zweiten Rahmenelement des Rahmens (32) mittels des Steuersystems (37) verstellbar ist;
wobei das Steuersystem (37) umfasst
- die Hydraulikzylinder (38, 39; 6.1, ..., 6.m; 70), die dafür eingerichtet sind, die relative Position zwischen den ersten und zweiten Rahmenelementen zu verstellen, wobei die Hydraulikzylinder (38, 39; 6,1, ..., 6.m; 70) jeweils versehen sind mit
- einer Zylinderkammer;
- einer vorderen Unterkammer und einer hinteren Unterkammer, welche jeweils in der Zylinderkammer bereitgestellt und durch eine innere Zylinderwand getrennt sind; und
- eine Kolbenstange, die sich beweglich durch ein vorderes Ende des Hydraulikzylinders und die innere Zylinderwand erstreckt, und an welcher ein vorderer Kolben und ein hinterer Kolben bereitgestellt sind, wobei der vordere Kolben in der vorderen Unterkammer und der hintere Kolben in der hinteren Unterkammer bereitgestellt ist, wodurch der vordere Kolben die vordere Unterkammer in Bezug auf die innere Wand in eine proximale vordere Unterkammer und eine distale vordere Unterkammer teilt, und der hintere Kolben die hintere Unterkammer in Bezug auf die innere Wand in eine proximale hintere Unterkammer und eine distale hintere Unterkammer teilt.

5. Landwirtschaftliche Maschine nach mindestens einem der vorstehenden Ansprüche, ferner umfassend mindestens eines
- eines Transportgestells aufweisend Transporträder (34) und in der Höhe verstellbar mittels des Steuersystems (37);
- eines Tiefensteuerelements bereitgestellt an einem Vorderteil des Rahmens (32) und verstellbar mittels des Steuersystems (37);
- eines vorderen Einstellrads (35) bereitgestellt an einem Vorderteil des Rahmens (32) und verstellbar mittels des Steuersystems (37); und
- eines Bodenwalzenrads (36), welches wahlweise hinter den Arbeitswerkzeugen (31) anzuordnen und in der Höhe mittels des Steuersystems (37) verstellbar ist.

6. Landwirtschaftliche Maschine nach mindestens einem der vorstehenden Ansprüche, ferner umfassend erste Hydraulikzylinder und zweite Hydraulikzylinder.

7. Landwirtschaftliche Maschine nach Anspruch 6, ferner umfassend
- einen ersten hydraulischen Steuerkreis, der für die Arbeitspositionssteuerung der Funktionselemente durch die ersten Hydraulikzylinder eingerichtet ist; und
- einen zweiten hydraulischen Steuerkreis, der für die Arbeitspositionssteuerung der Funktionselemente durch die zweiten Hydraulikzylinder eingerichtet ist, wobei der zweite hydraulische Steuerkreis getrennt vom ersten hydraulischen Steuerkreis betreibbar ist.

8. Landwirtschaftliche Maschine nach mindestens einem der vorstehenden Ansprüche, ferner umfassend
- eine Zugstange (40) bereitgestellt am Rahmen (32); und
- einen Zugstangen-Hydraulikzylinder, der mit dem Steuersystem (37) bereitgestellt ist, wobei der Zugstangen-Hydraulikzylinder für die Traktionsregelung eingerichtet ist, um eine auf einen oder mehrere Anhängepunkte der Zugstange (40) ausgeübte Last einzustellen, wenn die Zugstange (40) an dem einen oder den mehreren Anhängepunkten mit einer Zugmaschine (42) verbunden ist.

9. Landwirtschaftliche Maschine nach mindestens einem der vorstehenden Ansprüche, ferner umfassend eine Versatzsteuerung bereitgestellt mit dem Steuersystem (37), wobei die Versatzsteuerung dafür eingerichtet ist, einen Versatz zwischen einer vorderen Höhenposition, welche durch die vorderen Hydraulikzylinder (39) erzielt wird, und einer hinteren Höhenposition, welche durch die hinteren Hydraulikzylinder (39) erzielt wird, zu steuern.

10. Landwirtschaftliche Maschine nach mindestens einem der vorstehenden Ansprüche, bezugnehmend auf die Ansprüche 3 und 6, wobei
- der Rahmen (32) mindestens zwei Rahmenabschnitte aufweist, die nebeneinander in einer Richtung quer zu einer Fahrrichtung bereitgestellt sind; und
- jeder der mindestens zwei Rahmenabschnitte mit den Arbeitswerkzeugen (32), mindestens einem der ersten Hydraulikzylinder und mindestens einem der zweiten Hydraulikzylinder versehen ist.

11. Landwirtschaftliche Maschine nach mindestens einem der vorstehenden Ansprüche, ferner umfassend ein Benutzersteuerterminal, das funktional mit dem Steuersystem (37) verbunden ist, wobei das Benutzersteuerterminal dafür eingerichtet ist, Benutzereingaben für eine Benutzereinstellung von Steuerparametern, welche durch das Steuersystem (37) anzuwenden sind, zu empfangen.

12. Landwirtschaftliche Maschine nach mindestens einem der vorstehenden Ansprüche, ferner umfassend eines oder mehrere der folgenden Sensorelemente:
- einen ersten Drucksensor, der dafür eingerichtet ist, eine Belastungskraft auf den einen oder die mehreren Anhängepunkte der Zugstange (40) zu erfassen, wenn die Zugstange (40) mit der Zugmaschine (42) verbunden ist; und
- einen zweiten Drucksensor, der dafür eingerichtet ist, eine Belastungskraft auf das Tiefensteuerelement und/oder das vordere Einstellrad (35) zu erfassen.

13. Landwirtschaftliche Maschine nach mindestens einem der vorstehenden Ansprüche, ferner umfassend einen Positionssensor bereitgestellt an mindestens einem der Hydraulikzylinder (38, 39; 6.1, ..., 6.m; 70), wobei der Positionssensor dafür eingerichtet ist, Positionssensorsignale für den vorderen und/oder den hinteren Kolben in den Zylinderkammern des Hydraulikzylinders zu erfassen.

14. Verfahren zum Betreiben einer landwirtschaftlichen Maschine aufweisend
- einen Rahmen;
- ein Steuersystem (37) aufweisend eine Mehrzahl Hydraulikzylindern (38, 39; 6.1, ..., 6.m); und
- eine Mehrzahl von Funktionselementen bereitgestellt auf dem Rahmen (32) und beweglich in Arbeitspositionen durch die Mehrzahl von Hydraulikzylindern (38, 39; 6.1, ..., 6.m);
wobei
- das Steuersystem (37), mittels einer Synchronisierungsschaltung (46; 61; 81), welche mit jedem der Mehrzahl von Hydraulikzylindern (38, 39; 6.1, ..., 6.m; 70) aus der Mehrzahl von Hydraulikzylindern (38, 39; 6.1, ..., 6.m; 70) verbunden ist, die Bewegung sämtlicher der Mehrzahl von Hydraulikzylindern (38, 39; 6.1, ..., 6.m; 70) synchronisiert; und
- den Unterkammern der Hydraulikzylinder durch eine oder mehrere Versorgungsleitungen unter Druck stehende Flüssigkeit bereitstellt, wobei die eine oder die mehreren Versorgungsleitungen die Unterkammern mit der Hydraulikfluidquelle und der Hydraulikflüssigkeitspumpvorrichtung verbindet und getrennt von der Synchronisierungsschaltung bereitgestellt ist/sind.

## Revendications

1. Machine agricole comprenant :
- un châssis (32) ;
- un système de commande (37) ayant une pluralité de vérins hydrauliques (38, 39 ; 6.1, ..., 6.m) ; et
- une pluralité d'éléments fonctionnels fournis sur le châssis (32) et mobiles dans des positions de travail grâce à la pluralité des vérins hydrauliques (38, 39 ; 6.1, ..., 6.m) ;
dans laquelle
- le système de commande (37) comprend un circuit de synchronisation (46 ; 61 ; 81) se connectant à chacun de la pluralité des vérins hydrauliques (38, 39 ; 6.1, ..., 6.m ; 70) de la pluralité des vérins hydrauliques (38, 39 ; 6.1, ..., 6.m ; 70), le circuit de synchronisation (46 ; 61 ; 81) étant configuré pour synchroniser le mouvement de l'ensemble de la pluralité des vérins hydrauliques (38, 39 ; 6.1, ..., 6.m ; 70) ;
- **caractérisé en ce que** les sous-chambres des vérins hydrauliques (38, 39 ; 6.1, ..., 6.m ; 70) sont raccordées à la source de fluide hydraulique et au dispositif de pompe de fluide hydraulique via une ou plusieurs lignes d'alimentation (44, 45) fournies séparément du circuit de synchronisation (46 ; 61 ; 81).

2. Machine agricole selon la revendication 1, dans laquelle les vérins hydrauliques (38, 39 ; 6.1, ..., 6.m ; 70) sont dotés de l'un parmi un design de vérin à trois chambres et un design de vérin à quatre chambres.

3. Machine agricole selon l'une au moins des revendications précédentes, dans laquelle les éléments fonctionnels comprennent au moins les uns parmi des outils de travail (31) et des éléments de châssis du châssis (32).

4. Machine agricole selon la revendication 3, dans laquelle les outils de travail (31) sont fournis sur un premier élément de châssis du châssis (32), les outils de travail (31) étant configurés pour se mettre en prise avec le sol et/ou un produit agricole dans une pluralité de positions de travail contrôlées par le système de commande (37) dans un mode de commande, dans laquelle, pour localiser les outils de travail (31) dans la pluralité des positions de travail, une position du premier élément de châssis par rapport à un deuxième élément de châssis du châssis (32) est réglable au moyen du système de commande (37) ;
dans laquelle le système de commande (37) comprend
- les vérins hydrauliques (38, 39 ; 6.1, ..., 6.m ; 70) configurés pour régler la position relative entre les premier et deuxième éléments de châssis, dans laquelle les vérins hydrauliques (38, 39 ; 6.1, ..., 6.m ; 70) sont chacun dotés
- d'une chambre de vérin ;
- d'une sous-chambre avant et d'une sous-chambre arrière fournies toutes les deux dans la chambre de vérin et séparées par une paroi interne de vérin ; et
- d'une tige de piston s'étendant de manière mobile à travers une extrémité avant du vérin hydraulique et la paroi interne de vérin et sur laquelle un piston avant et un piston arrière sont fournis, dans laquelle le piston avant est fourni dans la sous-chambre avant et le piston arrière est fourni dans la sous-chambre arrière, le piston avant divisant ainsi la sous-chambre avant, par rapport à la paroi interne, en une sous-chambre avant proximale et une sous-chambre avant distale, et le piston arrière divisant la sous-chambre arrière, par rapport à la paroi interne, en une sous-chambre arrière proximale et une sous-chambre arrière distale.

5. Machine agricole selon l'une au moins des revendications précédentes, comprenant en outre au moins l'un/une parmi
- un châssis de transport ayant des roues de transport (34) et réglable en hauteur au moyen du système de commande (37) ;
- un élément de contrôle de profondeur fourni sur une partie avant du châssis (32) et réglable au moyen du système de commande (37) ;
- une roue de jauge avant (35) fournie sur une partie avant du châssis (32) et réglable au moyen du système de commande (37) ; et
- une roue à rouleau de terrain (36) à placer optionnellement à l'arrière des outils de travail (31) et réglable en hauteur au moyen du système de commande (37).

6. Machine agricole selon l'une au moins des revendications précédentes, comprenant en outre des premiers vérins hydrauliques et des deuxièmes vérins hydrauliques.

7. Machine agricole selon la revendication 6, comprenant en outre
- un premier circuit de réglage hydraulique configuré pour le contrôle de la position de travail des éléments fonctionnels par les premiers vérins hydrauliques ; et
- un deuxième circuit de réglage hydraulique configuré pour le contrôle de la position de travail des éléments fonctionnels par les deuxièmes vérins hydrauliques, le deuxième circuit de réglage hydraulique pouvant être actionné séparément du premier circuit de réglage hydraulique.

8. Machine agricole selon l'une au moins des revendications précédentes, comprenant en outre :
- une barre d'attelage (40) fournie sur le châssis (32) ; et
- un vérin hydraulique de barre d'attelage fourni avec le système de commande (37), le vérin hydraulique de barre d'attelage étant configuré, pour le contrôle de la traction, pour régler une charge s'exerçant sur un ou plusieurs points d'attache de la barre d'attelage (40) lorsque la barre d'attelage (40) est reliée à un tracteur (42) au niveau des un ou plusieurs points d'attache.

9. Machine agricole selon l'une au moins des revendications précédentes, comprenant en outre un dispositif de contrôle de décalage fourni avec le système de commande (37), le dispositif de contrôle de décalage étant configuré pour contrôler un décalage entre une position en hauteur avant imposée par les vérins hydrauliques avant (38) et une position en hauteur arrière imposée par les vérins hydrauliques arrières (39).

10. Machine agricole selon l'une au moins des revendications précédentes, faisant référence aux revendications 3 et 6, dans laquelle
- le châssis (32) a au moins deux sections de châssis fournies de manière adjacente l'une de l'autre dans une direction transversale à une direction de déplacement ; et
- chacune des au moins deux sections de châssis est dotée des outils de travail (32), d'au moins l'un parmi des premiers vérins hydrauliques et d'au moins l'un parmi des deuxièmes vérins hydrauliques.

11. Machine agricole selon l'une au moins des revendications précédentes, comprenant en outre un terminal de commande d'utilisateur connecté fonctionnellement au système de commande (37), le terminal de commande d'utilisateur étant configuré pour recevoir une entrée d'utilisateur pour un réglage d'utilisateur de paramètres de contrôle à appliquer par le système de commande (37).

12. Machine agricole selon l'une au moins des revendications précédentes, comprenant en outre un ou plusieurs des éléments de détection suivants :
- un premier capteur de pression configuré pour détecter une force de charge sur ce ou ces points d'attache de la barre d'attelage (40) lorsque la barre d'attelage (40) est reliée au tracteur (42) ; et
- un deuxième capteur de pression configuré pour détecter une force de charge sur au moins l'un parmi un élément de contrôle de profondeur et la roue de jauge avant (35).

13. Machine agricole selon l'une au moins des revendications précédentes, comprenant en outre un capteur de position fourni sur au moins l'un des vérins hydrauliques (38, 39 ; 6.1, ..., 6.m ; 70), le capteur de position étant configuré pour la détection des signaux de capteur de position pour au moins l'un parmi les pistons avant et arrière dans les chambres de vérin du vérin hydraulique.

14. Procédé pour faire fonctionner une machine agricole ayant
- un châssis,
- un système de commande (37) ayant une pluralité de vérins hydrauliques (38, 39 ; 6.1, ..., 6.m) ; et
- une pluralité d'éléments fonctionnels fournis sur le châssis (32) et mobiles dans des positions de travail grâce à la pluralité de vérins hydrauliques (38, 39 ; 6.1, ..., 6.m) ;
dans laquelle
- le système de commande (37), grâce à un circuit de synchronisation (46 ; 61 ; 81) se connectant à chacun de la pluralité des vérins hydrauliques (38, 39 ; 6.1, ..., 6.m ; 70) de la pluralité des vérins hydrauliques (38, 39 ; 6.1, ..., 6.m ; 70), synchronise le mouvement de l'ensemble de la pluralité des vérins hydrauliques (38, 39 ; 6.1, ..., 6.m ; 70) ; et
- la fourniture de fluide sous pression à des sous-chambres des vérins hydrauliques via une ou plusieurs lignes d'alimentation, ce ou ces lignes d'alimentation raccordant les sous-chambres à la source de fluide hydraulique et au dispositif de pompe de fluide hydraulique et étant fournies séparément du circuit de synchronisation.
